# FASCICULE DE BREVET EUROPEEN

(11) **EP 1 445 332 B1**
(45) Date de publication et mention de la délivrance du brevet: **27.04.2005**
(21) Numéro de dépôt: 04290239.5
(22) Date de dépôt: 30.01.2004
(51) Int. Cl.: C12Q 1/68

(54) **Séquences spécifiques d'E. Coli 0145, et leurs utilisations**
E. coli 0145-spezifische Sequenzen und ihre Verwendung
E. coli 0145 specific sequences and their uses

(30) Priorité: 06.02.2003 FR 0301436
(43) Date de publication de la demande: 11.08.2004
(73) Titulaire: Agence Française de Securité Sanitaire des Aliments, 94701 Maisons-Alfort Cedex (FR)
(72) Inventeur: Fach, Patrick, 94000 Creteil (FR); Perelle, Sylvie, 94220 Charenton-le-Pont (FR); Dilasser, Françoise, 91290 La Norville (FR); Grout, Joel, 93270 Sevran (FR)
(74) Mandataire: Vialle-Presles, Marie José

(56) Documents cités:
- WO-A-02/12538
- JENKINS C ET AL: "Serodiagnosis of infection with Verocytotoxin-producing Escherichia coli." JOURNAL OF APPLIED MICROBIOLOGY, vol. 86, no. 4, avril 1999 (1999-04), pages 569-575, XP002256134 ISSN: 1364-5072
- PERELLE S ET AL: "Development of a 5'-nuclease PCR assay for detecting Shiga toxin-producing Escherichia coli O145 based on the identification of an 'O-island 29' homologue." JOURNAL OF APPLIED MICROBIOLOGY, vol. 94, no. 4, avril 2003 (2003-04), pages 587-594, XP002256135 ISSN: 1364-5072

## Description

La présente invention est relative à de nouveaux marqueurs de pathogénicité *d'Escherichia coli*, utilisables notamment pour la détection des *Escherichia coli* pathogènes de sérotype O145 productrices de shiga-toxines.

*Escherichia coli* (*E*. *coli*) est une bactérie ubiquitaire, fréquemment isolée dans les laboratoires de microbiologie médicale ou alimentaire, et présente à l'état normal dans la flore intestinale de l'homme. Certaines souches sont toutefois pathogènes, et associées à de nombreuses infections intestinales et non-intestinales. Elles peuvent être transmises notamment par l'eau et l'alimentation, et en particulier les produits frais d'origine animale (viande, lait cru et produits laitiers à base de lait cru).

L'identification des différentes souches d'*E. coli* repose de façon classique, sur le typage sérologique de leurs antigènes O (antigène somatique) et H (antigène flagellaire) par des méthodes immunologiques.

Les sérotypes d'*E. coli* pathogènes les plus courants ont été classés en 5 groupes principaux, en fonction de leur mécanisme d'infection du tube digestif. Notamment, le groupe des VTEC « *verotoxin producing E. coli* » ou STEC « *shiga-toxin producing E. coli* », regroupe les *E. coli* produisant des cytotoxines dénommées *« Shiga-like toxins* », du fait de leur analogie avec la toxine sécrétée par *Shigella dysenteriae* de type 1, ou également vérotoxines (VT), du fait de leur activité toxique sur les cellules Véro.

Les souches d'*E. coli* productrices de shiga-toxines (STEC) sont des microorganismes de plus en plus reconnus comme responsables d'infections d'origine alimentaire (GANNON et al., J. Clin. Microbiol., 31, 1268-1274, 1993 ; BEGUM et al., J. Clin. Microbiol., 31, 3153-3156, 1993). Chez l'homme, l'infection par des STEC se traduit par des symptômes plus ou moins graves qui peuvent aller d'une simple diarrhée, à une colite hémorragique (CH), jusqu'à un syndrome hémolytique et urémique (SHU) ou un purpura thrombotique thrombocytopénique (PTT), et peut parfois être fatale (GRIFFIN et al., Epidemiol. Rev., 13, 60-98, 1991 ; NATARO et KAPER, Microbiol. Immunol., 42, 371-376, 1998 ; PATON et PATON, Clin. Microbiol. Rev., 11, 450-479, 1998).

Un grand nombre de ces infections a été attribué aux STEC de sérotype 0157:H7 ; il a toutefois été observé que divers autres sérotypes pouvaient être impliqués dans des infections gastro-intestinales à STEC. Parmi ceux-ci, le sérotype 0145 est l'un des sérotypes non-0157 les plus communs à travers le monde (BEUTIN et al., Medical Microbiol. Immunol., 183, 13-21, 1994 ; BEUTIN et al., Emerging Inf. Dis., 4, 635-639, 1998 ; GOLDWATER et BETTELHEIM, J. Medical Microbiol., 47, 1039-1045, 1998 ; GOLDWATER et BETTELHEIM, Scandinavian J. Inf. Dis., 32, 385-394, 2000 ; EKLUND et al., J. Clin. Microbiol., 39, 2829-2834, 2001).

La pathogénicité des STEC résulte principalement des shiga-toxines produites par ces bactéries (CALDERWOOD et al., 1987, Proc. Nat. Acad. Sci. 84 :4364-4368). Cependant, d'autres facteurs de virulence pourraient également jouer un rôle dans le pouvoir pathogène de ces colibacilles (LAW, Journal of Applied Microbiology, 88, 729-745, 2000).

Pour prévenir la diffusion des pathologies associées aux STEC, il est nécessaire de pouvoir déterminer rapidement le sérotype concerné. En effet, la connaissance de de ce sérotype permet de rechercher un lien épidémiologique entre différents cas observés, ainsi qu'avec des souches isolées à partir d'aliments suspectés.

Des tests immunologiques de détection de l'antigène O ont été proposés (NATARO et KAPER, Clin. Microbiol. Rev., 11, 142-201, 1998) mais la plupart ne sont pas disponibles pour tous les sérogroupes. En outre on observe parfois des réactions croisées entre les antisérums anti-O (BORCZYK et al., Int. J. Food Microbiol., 4, 347-349, 1987; CHART et al., Epidemiol. Infect., 108, 77-85, 1992 ; SHIMADA et al., Curr. Microbiol., 25, 215-217, 1992).

Des méthodes d'amplification en chaîne par polymérase (PCR), permettant la détection spécifique de gènes codant des facteurs bactériens impliqués dans la virulence ont été proposées (NATARO et KAPER, Clin. Microbiol. Rev., 11, 142-201, 1998). Cependant, ces méthodes ne permettent pas de déterminer rapidement le sérotype impliqué.

Plusieurs équipes ont proposé un sérotypage O par PCR, en ciblant deux types de gènes de virulence. Les variations de séquence dans le locus d'effacement entérocytaire (LEE) (GANON et al., Journal of Clinical Microbiology 31, 1268-1274, 1993 ; LOUIE et al., Epidemiology and Infection 112, 449-461, 1994 ; LOUIE et al., Journal of Clinical Microbiology 36, 3375-3377,1998 ; ZHAO et al., FEMS Microbiology Letters 133, 35-39, 1995 ; MENG et al., International Journal of Food Microbiology 32, 103-113, 1996 ;. Letters in Applied Microbiology 24, 172-176, 1997), ou dans les gènes impliqués dans la biosynthèse de l'antigène O (locus rfb) (DEMARCHELIER et al., Journal of Clinical Microbiology 36,1801-1804, 1998 ; PATON et PATON, Clinical Microbiology Reviews 11, 450-479, 1998 ;. Infection and Immunity 67, 5930-5937, 1999 ; Journal of Clinical Microbiology 37, 3362-3365, 1999 ; WANG et REEVES, Infection and Immunity 66, 3545-3551, 1998 ; WANG et al., Journal of Clinical Microbiology 36, 3182-3187, 1998 ; Gene 270, 231-236, 2001 ; MAURER et al., Applied and Environmental Microbiology 65, 2954-2960, 1999 ; PERELLE et al., Journal of Applied Microbiology 93, 758-764, 2002) ont ainsi été utilisées pour définir des amorces pour la détection spécifique des sérogroupes O26, O91, O103, O104, O111, O113 et O157.

Récemment les inventeurs ont identifié dans des STEC de sérotype O157, la présence d'un insert d'acide nucléique représentant un nouveau locus (SIL_{O157}), absent des souches non-pathogènes (Demande PCT WO 02/12538 ; PERELLE et al., *J. Appl. Microbiol*., 93 (2), 250-260, 2002)

Ils ont également constaté la présence d'un locus similaire, inséré à la même position, chez des souches d'E. coli pathogènes, appartenant à des sérotypes autres que O157, en particulier chez les STEC de sérotype 0 55, 077, 079, et O145.

Les Inventeurs ont entrepris d'isoler le locus détecté chez les STEC de sérotype O145, dont la taille apparaissait supérieure à celle du locus SIL_{O157}, afin de déterminer s'il possédait des régions suffisamment spécifiques pour permettre la détection sélective de ce sérogroupe.

L'analyse du locus SIL_{O145}, a permis de mettre en évidence 4 cadres ouverts de lecture (ORFs) .
- L'ORF1 (SEQ ID NO: 1) code pour une adhésine/invasine putative IHP1-like de 436 acides aminés (SEQ ID NO: 2) présentant 71% d'identité et 84% de similarité avec la protéine IHP1 de la souche STEC O157:H7 (GenBank AAG54843).
- L'ORF2 (SEQ ID NO : 3) code pour une protéine IHP2 de 140 acides aminés (SEQ ID NO : 4) présentant 100% d'identité avec la protéine IHP2 de la souche STEC O157:H7 (GenBank AAG54842)
- L'ORF 3 (complémentaire de la SEQ ID NO : 5) code pour une protéine IHP3 de 120 acides aminés (SEQ ID NO : 6) présentant 100% d'identité avec la protéine IHP3 de la souche STEC O157:H7 (GenBank AAG54844).
- L'ORF 4 (SEQ ID NO : 7) code pour une protéine cytoplasmique putative (SEQ ID NO : 8) de 407 acides aminés ne présentant aucune homologie avec la souche STEC O157:H7, mais présentant 53% d'identité et 67% de similarité avec une protéine de *Salmonella Typhimurium* LT2 (GenBank AAL19611).

Les Inventeurs ont dans un premier temps, cherché à définir des sondes d'acide nucléique et amorces d'amplification à partir de la séquence de l'ORF 4, absente de O157. Toutefois, ils ont constaté que cette séquence présentait des régions d'homologie importante avec d'autres bactéries (notamment des *E*. *coli* de sérotypes O125 et O126), rendant impossible son utilisation pour la détection spécifique de O145.

Ils ont alors recherché si d'autres régions du locus SIL_{O145} pouvaient éventuellement être utilisées pour la détection spécifique des STEC O145. Ils sont parvenus à identifier une région de l'ORF1 du locus SIL_{O145} spécifique du sérotype O145, et permettant de définir des sondes et des amorces ne présentant de réactions croisées ni avec O157, ni avec les autres bactéries testées.

Ils ont ainsi conçu de nouveaux marqueurs utiles pour la détection de souches d'*E*. *coli* productrices de shiga-toxines de sérotype O145.

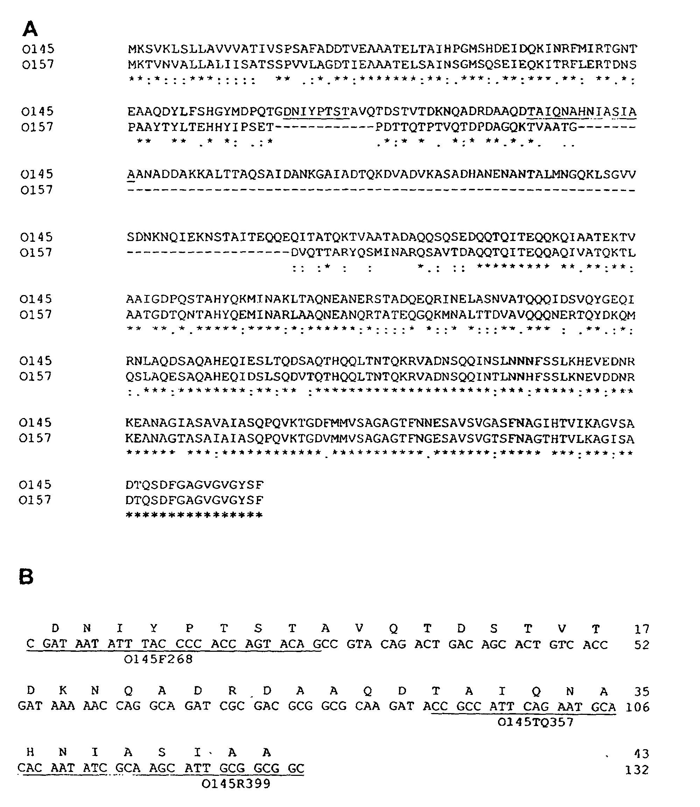

L'alignement des séquences polypeptidiques de l'adhésine putative (ORF1) du locus SIL_{O145} et de l'adhésine putative de la souche STEC O157 (O157) est représenté sur la Figure 1A.

L'adhésine de la souche STEC O145 présente notamment, par rapport à celle de O157, deux séquences additionnelles correspondant respectivement aux acides aminés 77-90 et 113-200 de l'adhésine de O145 (SEQ ID NO: 2). Les Inventeurs ont constaté que la séquence codant pour la région 77-200 de l'adhésine de O145, représentait une séquence-cible, permettant la détection sélective de souches d'*E. coli* productrices de shiga-toxines de sérotype O145, sans réactions croisées avec les souches STEC d'autres sérotypes, les souches d'*E. coli* non-STEC et les souches d'autres espèces bactériennes. Cette séquence est représentée dans la liste de séquences en annexe sous le numéro SEQ ID NO: 9.

La présente invention a pour objet l'utilisation d'un polynucléotide choisi dans le groupe constitué par :
a) le polynucléotide de séquence SEQ ID NO : 9, ou son complémentaire;
b) tout fragment d'au moins 15 pb, dudit polynucléotide a) ou son complémentaire ;
pour la détection spécifique de bactéries E. coli de sérotype 0145 productrices de shiga-toxines.

Les polynucléotides définis ci-dessus peuvent notamment constituer des sondes d'acide nucléique ou des amorces d'amplification utilisables pour la détection de bactéries *E*. *coli* de sérotype O145 productrices de shiga-toxines. Des sondes d'acide nucléique conformes à l'invention comprennent de préférence un fragment d'au moins 20 pb, de préférence 25 à 50 pb du polynucléotide de séquence SEQ ID NO : 9 ou de son complémentaire. Des amorces d'amplification conformes à l'invention comprennent de préférence de 15 à 30 pb du polynucléotide de séquence SEQ ID NO : 9 ou de son complémentaire.

A titre d'exemple non-limitatif d'amorces d'amplification conformes à l'invention, on citera le couple d'amorces constitué par les polynucléotides SEQ ID NO: 11 et SEQ ID NO:12, qui permet l'amplification d'un fragment de 132 pb à partir de la séquence SEQ ID NO: 1.

A titre d'exemple non limitatif de sonde d'acide nucléique conforme à l'invention, on citera le polynucléotide de séquence SEQ ID NO: 13.

Le fragment de 132 pb amplifié par les amorces SEQ ID NO: 11 et SEQ ID NO:12 est représenté dans la liste des séquences en annexe sous le numéro SEQ ID NO:10, la position des amorces et de la sonde interne SEQ ID NO : 13 par rapport à ce fragment est représentée sur la Figure 1B.

La présente invention a également pour objet un procédé de détection d'*E. coli* productrices de shiga-toxines de sérotype O145, caractérisé en ce qu'il comprend :
- la mise en présence d'ADN d'un échantillon biologique susceptible de contenir ladite bactérie avec au moins un polynucléotide conforme à l'invention, en conditions permettant une hybridation sélective entre ledit polynucléotide et sa séquence cible, si celle-ci est présente dans ledit ADN ;
- la détection de ladite hybridation.

La détection de l'hybridation peut s'effectuer par tous moyens connus en eux-mêmes de l'homme de l'art.

Selon un mode de mise en oeuvre préféré du procédé de dépistage conforme à l'invention comprend au moins une étape d'amplification en chaîne par polymérase, comprenant :
- la mise en présence de l'ADN à tester avec au moins un couple d'amorces conformes à l'invention ;
- la détection du produit d'amplification, si celui-ci est présent.

Selon une disposition préférée de ce mode de mise en oeuvre, le procédé conforme à l'invention comprend une étape d'amplification en chaîne par polymérase quantitative (par exemple 5'-nucléase PCR). Dans ce cas, la détection du produit d'amplification s'effectue par hybridation de celui-ci avec une sonde interne.

Par exemple, les polynucléotides SEQ ID NO: 11 et et SEQ ID NO: 12 peuvent avantageusement être utilisés comme amorces et le polynucléotide SEQ ID NO : 13 comme sonde interne.

La présente invention sera mieux comprise à l'aide du complément de description qui va suivre, qui se réfère à des exemples non-limitatifs illustrant la caractérisation du locus SIL_{O145} et l'utilisation de séquences spécifiques de ce locus pour la détection sélective de bactéries *Escherichia coli* O145 productrices de shiga-toxines.

### EXEMPLE 1 : IDENTIFICATION ET CARACTERISATION DU LOCUS SIL_{O145}

Une souche de STEC de sérotype O145 (souche *d'E. coli* VTH34) est cultivée dans du milieu TSB (*Tryptone Soy Broth*), à 37°C, pendant une nuit et l'ADN bactérien est extrait à l'aide de la matrice INSTAGENE™ (BIO-RAD, Marnes-la-Coquette, France), en suivant les instructions du fabricant. La préparation d'ADN est conservée à -20°C jusqu'à utilisation.

La réaction PCR est réalisée à l'aide du GENE-AMP PCR SYSTEM 9700 (Applied Biosystems, Courtaboeuf, France), et d'amorces obtenues par les techniques classiques de synthèse oligonucléotidique.

Le couple d'amorces suivant : est utilisé pour amplifier une séquence d'acide nucléique à partir de l'ADN bactérien extrait de la souche de STEC de sérotype O145 (souche VTH34), à l'aide du EXPAND™ HIGH FIDELITY PCR SYSTEM (Roche Diagnostics, Meylan, France), en suivant les instructions du fabricant.

L'amplification PCR est réalisée dans les conditions suivantes : une étape initiale de dénaturation à 94°C pendant 2 minutes est suivie de 10 cycles, alternant une étape à 94°C pendant 10 secondes, une étape à 68°C pendant 30 secondes et une étape à 68°C pendant 4 minutes, suivis de 20 cycles avec une étape d'extension augmentée de 5 secondes à chaque cycle, et une étape finale d'élongation à 72°C pendant 7 minutes. Le produit d'amplification est ensuite séquencé.

La séquence nucléotidique de ce produit d'amplification (locus SIL_{O145}), est homologue de celle du locus SIL_{O157}, obtenue à partir de la souche de STEC de sérotype O157. Elle comprend 4255 pb soit 1621 pb de plus que celle du locus SIL_{O157}.

Cette séquence nucléotidique contient 3 ORFs homologues des ORFs du locus SILO₁₅₇ de la souche STEC O157 et un ORF supplémentaire à son extrémité 3'. Les localisations et les caractéristiques des 4 ORFs sont présentées dans le Tableau 1 ci-dessous :

**Tableau 1**

| Position dans STEC O145 | Aminoacides (nbre) | Protéine similaire (N° d'accession) | % identité/similarité (nbre aminoacides) | Fonction putative des protéines |
|---|---|---|---|---|
| ORF1 : 1150-2460 | 436 | *E. coli* O-157:H7 (AAG54843) | 71/84 (200) | Adhésine/invasine |
| ORF2 : 2561-2983 | 140 | *E. coli* O-157:H7 (AAG54842) | 100 (140) | Inconnue |
| ORF4 : 3275-4498 | 407 | S. *typhimurium* LT2 (AAL19611) | 53/67 (412) | Protéine cytoplasmique |
| ORF3 : 784-422^{c} | 120 | *E. coli* O-157:H7 (AAG54844) | 100 (120) | Inconnue |

| | | | | |
|---|---|---|---|---|
| ^{c}: brin complémentaire | | | | |

Parmi les 3 ORFs homologues, deux codent pour des protéines présentant 100% d'identité avec les protéines putatives de la souche STEC O157:H7.

En revanche les protéines (adhésine/invasine putative) trouvées dans les loci SIL_{O157} et SIL_{O145} des souches STEC O157 et STEC 0145, respectivement, présentent des divergences mises en évidence suite à l'alignement des séquences d'aminoacides des adhésines putatives des souches STEC O145 (O145) et STEC O157 (O157) tel que représenté dans la Figure 1A (« * »=résidu identique, « : »=résidu très similaire ; « . »=résidu moins similaire). Avec 436 aminoacides, l'adhésine de la souche STEC 0145 est plus longue que l'adhésine de 338 aminoacides de la souche STEC O157, du fait de deux séquences additionnelles dans sa région N-terminale entre les acides aminés 77-90 et 113-200, respectivement. Leurs extrémités C-terminales présentent une similarité élevée, alors que leurs extrémités N-terminales présentent quelques divergences.

La quatrième protéine codée par l'extrémité 3' additionnelle du locus SIL_{O145} (ORF4) ne présente aucune homologie avec une protéine codée par un ORF du locus SIL_{O157} de la souche STEC 0157:H7, mais elle présente 53% d'identité globale avec la protéine cytoplasmique putative de *Salmonella Typhimurium* LT2.

### EXEMPLE 2 : DETECTION DE LA PRESENCE DE L'ORF CODANT POUR LA PROTEINE IHP1-LIKE DANS DES SOUCHES STEC

### 1. Extraction de l'ADN bactérien

195 souches bactériennes : 12 souches STEC de sérotype 0145 ; 124 souches STEC d'autres sérotypes : O3(2), O4(1), O5(7), O6(9), O8(1), O15(1), O22(2), O26(8), O45(1), O53(2), O55(1), O76(3), O77(3), O79(1), O86(1), O88(1), O91(6), O103(7), O110(2), O111(11), O113(3), O117(2), O118(2), O125(1), O126(1), O128(1), O136(2), O138(1), O139(2), O141(1), O147(1), O157(37)] ; 25 souches non productrices de shiga-toxines (non-STEC) [026(2), O55(3), O103(1), O111(2), O125(1), O126(1), O127(1), O128(1), O157(13)] ; et 34 souches d'autres espèces bactériennes *Hafnia alvei* (5), *Enterobacter sakasaki* (1), *Salmonella enterica* de sérotype O30 (8), *Salmonella indiana* (1), *Salmonella montevideo* (1), *Salmonella enterica subsp. arizonae* (1), *Salmonella* *seftenberg* (1), *Salmonella hadar* (1), *Salmonella enteritidis* (1), *Salmonella anatum* (1), *Salmonella salford* (1), *Salmonella cerro* (1), *Salmonella fresno* (1), *Salmonella kisubi* (1), *Enterococcus faecalis* (1), *Shigella boydii* (1), *Klebsiella pneumoniae* (1), *Staphylococcus aureus* (1), *Citrobacter freundii* (1), *Yersinia enterocolitica* (1), *Proteus mirabilis* (1), *Campylobacter jejuni* (1), *Listeria monocytogenes* (1) provenant de différents laboratoires sont utilisées. Le nombre de souches pour chaque sérotype ou espèce bactérienne est indiqué entre parenthèses. Les bactéries sont cultivées dans du milieu TSB (*Tryptone Soy Broth*), à 37°C, pendant une nuit et l'ADN bactérien est extrait à l'aide de la matrice INSTAGENE™ (BIO-RAD, Marnes-la-Coquette, France), en suivant les instructions du fabricant.

Les préparations d'ADN sont conservés à -20°C jusqu'à utilisation.

### 2. Détection par 5'-nucléase PCR

Le couple d'amorces suivant : et la sonde interne TaqMan® (O145TQ357) de séquence SEQ ID NO : 13 : sont préparés à partir de la séquence nucléotidique de l'ORF1 du locus SIL_{O145} de la souche STEC O145.

Les séquences et localisations des amorces spécifiques de STEC O145 et de la sonde sont représentées sur la Figure 1.

Les amplifications sont réalisées avec un mélange réactionnel de 20 µl contenant une concentration 1X d'un mélange LIGHTCYCLER-FASTSTART DNA MASTER HYBRIDIZATION PROBES MIX (Roche Diagnostics), 5 mM de MgCl₂, 500 nM de chacune des amorces, 200 nM de la sonde TaqMan®, et 2 µl d'ADN bactérien.

Les réactions d'amplifications sont réalisées dans l'appareil LIGHTCYCLER (Roche Diagnostics), dans les conditions présentées dans le Tableau 2 ci-dessous :

**Tableau 2**

| Etapes | Température (°C) | Durée (secondes) | Pente (°C/secondes) | Mode d'acquisition |
|---|---|---|---|---|
| Dénaturation | 95 | 480 | 20 | Aucun |
| Amplification (40 cycles) | 95 | 10 | 20 | Aucun |
| | 60 | 30 | 20 | Simple |
| Hybridation | 60 | 30 | 20 | Aucun |

Sur la base du polymorphisme des gènes codant pour l'adhésine putative des loci SIL_{O157} et SIL_{O145} des souches STEC O157 et 0145, respectivement, une TaqMan® PCR est réalisée pour détecter spécifiquement la présence du gène codant pour cette adhésine putative dans des souches d' *E. coli*.

### a) Spécificité de la détection

La spécificité de détection est testée vis-à-vis d'un panel d'échantillons bactériens provenant de 136 souches de STEC comprenant 33 sérotypes, 25 souches d'*E. coli* non productrice de shiga-toxines comprenant 9 sérotypes et 34 souches bactériennes d'autres espèces.

Les résultats de la PCR montrent l'amplification d'un fragment d'acide nucléique de 132 pb dans le cas des 12 souches de STEC de sérotype O145, démontrant la présence du gène codant pour l'adhésine IHP1-like dans leur ADN, alors qu'aucune amplification n'a lieu dans le cas des autres souches bactériennes. Les 12 échantillons positifs donnent des valeurs C_{T} (définies comme le cycle PCR où l'augmentation de fluorescence au-dessus de la ligne de base (bruit de fond) apparaît en premier) inférieures à 20, et un signal de fluorescence élevé, généralement supérieur à 1 (données non présentées).

### b) Sensibilité de la détection

La souche d'*E*. *coli* MOSBlue contenant le plasmide pMOSBlue est cultivée dans le milieu LB (*Luria Broth*) ou sur plaques TSA (*Tryptone Soy Agar*), à 37°C.

L'ADN plasmidique est purifié en utilisant le kit QIAGEN PLASMID KIT (Qiagen, Courtaboeuf, France).

Pour déterminer la limite de détection de la 5'-nucléase PCR, le produit de PCR de 132 pb (SEQ ID NO : 10) obtenu à partir de l'ADN bactérien extrait de la souche STEC O145 (souche d'*E. coli* VTH34) à l'aide des amorces O145F268 et O145R399, est cloné dans le plasmide pMOSBlue en utilisant le kit de clonage « pMOSBlue blunt ended » (Amersham Pharmacia Biotech, Saclay, France), en suivant les instructions du fabricant.

La concentration en ADN du plasmide recombinant pSP67 est estimée par fluorimètre.

La sensibilité de la détection est déterminée sur le fragment PCR de 132 pb cloné dans le vecteur pSP67. La concentration estimée de l'ADN non dilué de pSP67 est de 2,09 µg/µl, ce qui correspond à 6,4x10¹¹ molécules/µl. Ce nombre est calculé comme suit : 3019 pb (2887 pb + 132 pb) correspondent à 1,96x10⁶ g/mol ; 2,09 µg d'un plasmide de 3019 pb contiennent 1,06x10⁻¹² mol. En multipliant ce nombre par le nombre d'Avogadro (6x10²³), on obtient un nombre de molécules de 6,4x10¹¹ molécules/µl dans l'ADN plasmidique non dilué.

A partir de la détermination de la concentration en molécules dans l'échantillon plasmidique initial, des séries de dilution par 5 de l'ADN du plasmide recombinant pSP67 sont utilisées pour déterminer la limite de détection de la 5'-nucléase PCR. Pour chacun des 11 échantillons, la fluorescence (F1/F2) est détectée en fonction du nombre de cycles de PCR effectués. 10 µl du produit d'amplification correspondant à chaque réaction de PCR, sont séparés par électrophorèse en gel d'agarose à 2% contenant du bromure d'éthidium, dans du tampon TBE (Tris-Borate-EDTA). Les fragments d'ADN amplifiés sont visualisés sous une lampe à ultraviolet.

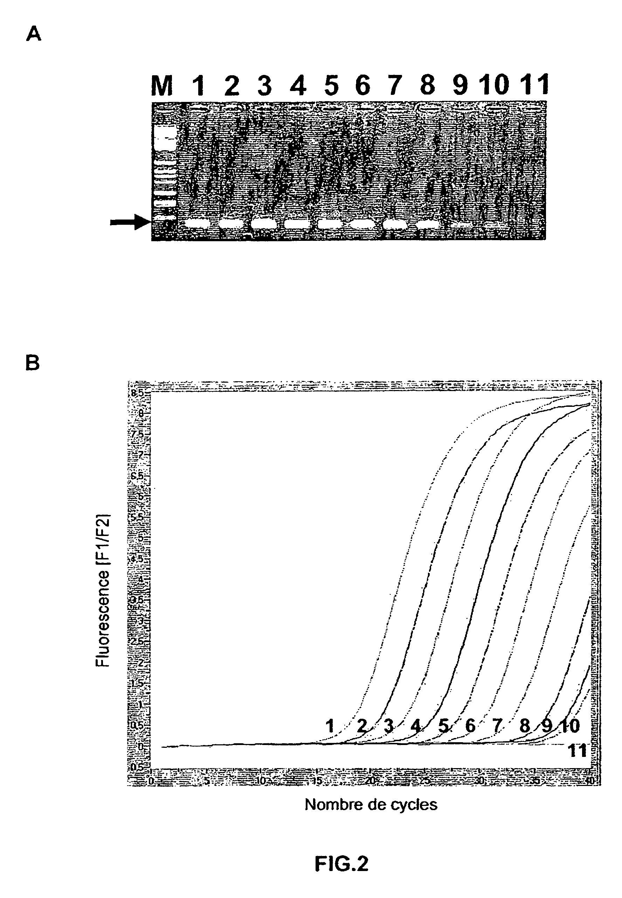

Les résultats sont représentés dans la Figure 2.

A : Gel d'électrophorèse : la piste M correspond au standard de poids moléculaire (Roche Diagnostics). Les pistes 1 à 11 correspondent respectivement aux produits d'amplification des échantillons 1 à 11. La flèche indique la position du fragment de PCR de 132 pb obtenu avec les amorces O145F268 et O145R399.

B : Les courbes d'amplification 1 à 11 correspondent respectivement à 10⁷, 2x10⁶, 4x10⁵, 8x10⁴, 15x10³, 3x10³, 625, 125, 25, 5 et 0 copies du plasmide recombinant pSP67, et représentent la fluorescence (F1/F2) détectée en fonction du nombre de cycles de PCR effectués.

Ces résultats montrent que la limite de détection de la 5'-nucléase PCR est de 5 copies.

La correspondance entre les valeurs C_{T} (définies comme le cycle PCR où l'augmentation de fluorescence au-dessus de la ligne de base (bruit de fond) apparaît en premier), et le nombre de copies du plasmide pSP67, est évaluée à partir des produits d'amplification 1 à 10 afin de déterminer l'efficacité de la PCR.

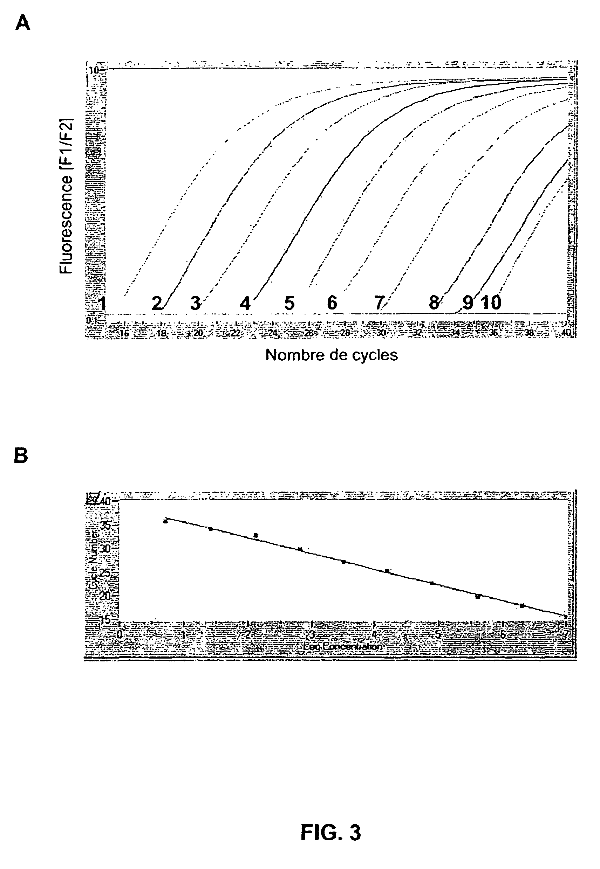

Les résultats sont présentés dans la Figure 3.

La Figure 3A représente les courbes d'amplification des échantillons 1 à 10. La Figure 3B représente la ligne droite obtenue par régression logarithmique des courbes 1 à 10 de la Figure 3A. Cette droite présente une pente de -3,33 correspondant à une efficacité de la réaction de PCR de 99% calculée selon la formule : [% d'efficacité = (10^{-1/pente} - 1) x 100].

La sensibilité de la réaction 5'-nucléase PCR a également été évaluée à partir d'ADN d'une série de dilutions au ½ d'une culture d'une nuit de la souche STEC 0145 VTH34. Elle est d'environ 1 CFU (colony forming unit).

## Revendications

1. Utilisation d'un polynucléotide choisi dans le groupe constitué par :
a) le polynucléotide de séquence SEQ ID NO : 9, ou son complémentaire;
b) tout fragment d'au moins 15 pb, dudit polynucléotide
a) ou son complémentaire ;
pour la détection spécifique de bactéries E. *coli* de sérotype O145 productrices de shiga-toxines.

2. Couple d'amorces d'amplification pour la détection de bactéries *E. coli* de sérotype O145 productrices de shiga-toxines, **caractérisées en ce que** chacune desdites amorces comprend 15 à 30 pb du polynucléotide de séquence SEQ ID NO : 9 ou de son complémentaire.

3. Couple d'amorces d'amplification selon la revendication 2, **caractérisé en ce qu'**il est constitué par les amorces SEQ ID NO: 11 et SEQ ID NO:12.

4. Sonde d'acide nucléique pour la détection de bactéries *E*. *coli* de sérotype 0145 productrices de shiga-toxines, **caractérisée en ce qu'**elle comprend un fragment d'au moins 20 pb du polynucléotide de séquence SEQ ID NO : 9 ou de son complémentaire.

5. Sonde d'acide nucléique selon la revendication 4, répondant à la séquence SEQ ID NO: 13.

6. Procédé de détection de bactéries E. *coli* de sérotype O145 productrices de shiga-toxines, **caractérisé en ce qu'**il comprend :
- la mise en présence d'ADN d'un échantillon biologique susceptible de contenir lesdites bactérie avec un couple d'amorces selon une quelconque des revendications 2 ou 3, et/ou avec une sonde d'acide nucléique selon une quelconque des revendications 4 ou 5, en conditions permettant une hybridation sélective entre ledit couple d'amorces ou ladite sonde, et sa séquence cible, si celle-ci est présente dans ledit ADN ;
- la détection de ladite hybridation.

7. Procédé selon la revendication 6, **caractérisé en ce qu'**il comprend au moins une étape d'amplification en chaîne par polymérase avec un couple d'amorces selon une quelconque des revendications 2 ou 3.

8. Procédé selon la revendication 7, **caractérisé en ce qu'**il comprend en outre la détection du produit d'amplification à l'aide d'une sonde selon une quelconque des revendications 3 ou 4.

## Patentansprüche

1. Verwendung eines Polynucleotids, das aus der Gruppe ausgewählt ist, die besteht aus:
a) dem Polynucleotid mit der Sequenz SEQ ID Nr. 9 oder seinem Komplement;
b) jedem Fragment mit mindestens 15 Basispaaren dieses Polynucleotids a) oder seines Komplements;
für die spezifische Erfassung von Shiga-Toxinen erzeugenden *E. Coli*-Bakterien des Serotyps 0145.

2. Amplifikationsinitiatorpaar für die Erfassung von Shiga-Toxinen erzeugenden *E. Coli-Bakterien* des Serotyps 0145, **dadurch gekennzeichnet, dass** jeder der Initiatoren 15 bis 30 Basispaare des Polynucleotids mit der Sequenz SEQ ID Nr. 9 oder seines Komplements umfasst.

3. Amplifikationsinitiatorpaar nach Anspruch 2, **dadurch gekennzeichnet, dass** es aus den Initiatoren SEQ ID Nr. 11 und SEQ ID Nr. 12 besteht.

4. Nucleinsäuredetektor zur Erfassung von Shiga-Toxinen erzeugenden *E. Coli*-Bakterien des Serotyps 0145, **dadurch gekennzeichnet, dass** er ein Fragment mit mindestens 20 Basispaaren des Polynucleotids mit der Sequenz SEQ ID Nr. 9 oder seines Komplements umfasst.

5. Nucleinsäuredetektor nach Anspruch 4, der auf die Sequenz SEQ ID Nr. 13 anspricht.

6. Verfahren zur Erfassung von Shiga-Toxinen erzeugenden *E. Coli*-Bakterien des Serotyps 0145, **dadurch gekennzeichnet, dass** es umfasst:
- Versehen der DNA einer biologischen Probe, die in der Lage ist, die Bakterien zu enthalten, mit einem Initiatorpaar nach einem der Ansprüche 2 oder 3, und/oder mit einem Nucleinsäuredetektor nach einem der Ansprüche 4 oder 5, unter Bedingungen, die eine selektive Hybridisierung zwischen dem Initiatorpaar oder dem Detektor und der Zielsequenz zulassen, wenn diese in der DNA vorhanden ist;
- Nachweisen der Hybridisierung.

7. Verfahren nach Anspruch 6, **dadurch gekennzeichnet, dass** es mindestens einen Schritt der Kettenamplifikation durch Polymerase mit einem Initiatorpaar nach einem der Ansprüche 2 oder 3 umfasst.

8. Verfahren nach Anspruch 7, **dadurch gekennzeichnet, dass** es darüber hinaus die Erfassung des Amplifikationsprodukts mittels eines Detektors nach einem der Ansprüche 3 oder 4 umfasst.

## Claims

1. Use of a polynucleotide selected from the group consisting of:
a) the polynucleotide of sequence SEQ ID NO : 9, or its complement;
b) any fragment of at least 15 bp, of said polynucleotide a) or its complement ;
for the specific detection of Shiga toxin-producing *E*. *coli* bacteria of serotype 0145.

2. Pair of amplification primers for the detection of Shiga toxin-producing E. coli bacteria of serotype 0145, **characterised in that** each of said primers comprises from 15 to 30 bp of the polynucleotide of sequence SEQ ID NO : 9 or of its complement.

3. Pair of amplification primers according to claim 2, **characterised in that** it is composed of the primers SEQ ID NO: 11 and SEQ ID NO: 12.

4. Nucleic acid probe for the detection of Shiga toxin-producing *E. coli* bacteria of serotype 0145, **characterised in that** it comprises a fragment of at least 20 bp of the polynucleotide of sequence SEQ ID NO : 9 or of its complement.

5. Nucleic acid probe according to claim 4 corresponding to sequence SEQ ID NO: 13.

6. Method of detecting Shiga toxin-producing *E*. *coli* bacteria of serotype O145, **characterised in that** it comprises:
- bringing DNA of a biological sample that is likely to contain said bacteria into contact with a pair of primers according to either claim 2 or claim 3 and /or with a nucleic acid probe according to either claim 4 or claim 5, under conditions permitting selective hybridization between said pair of primers or said probe and its target sequence ,if the latter is present in said DNA;
- detecting said hybridization.

7. Method according to claim 6, **characterised in that** it comprises at least one step of polymerase chain reaction using a pair of primers according to either claim 2 or claim 3.

8. Method according to claim 7, **characterised in that** it further comprises detection of the amplification product with the aid of a probe according to either claim 3 or claim 4.


### SEQUENCE LISTING

<110> AGENCE FRANCAISE DE SECURITE SANITAIRE DES ALIMENTS.
<120> SEQUENCES SPECIFIQUES D'E. COLI O145, ET LEURS UTILISATIONS
<130> MJPbv1159/4
<150> FR 03 01436
   <151> 2003-02-06
<160> 15
<170> PatentIn version 3.1
<210> 1
   <211> 1311
   <212> DNA
   <213> Escherichia coli
<220>
   <221> misc_feature
   <223> ORF1 du locus SILO145
<400> 1
   atgaaatctg taaagttgtc tttactggca gtcgttgttg ctaccattgt gagcccatct      60
   gcttttgcag atgatacggt tgaagcggca gcgacagaac tgacagcgat tcacccaggt     120
   atgtctcatg atgaaatcga tcaaaaaatc aatcgcttca tgattcggac aggtaataca     180
   gaagccgcgc aagattacct gttttctcat ggatatatgg acccacagac aggcgataat     240
   atttacccca ccagtacagc cgtacagact gacagcactg tcaccgataa aaaccaggca     300
   gatcgcgacg cggcgcaaga taccgccatt cagaatgcac acaatatcgc aagcattgcg     360
   gcggcaaatg cagatgacgc caaaaaagcg cttaccactg cgcaatctgc aattgatgcg     420
   aacaagggag ctattgctga tactcagaaa gatgtcgcag atgttaaagc aagtgcagat     480
   catgccaacg agaacgctaa taccgcgttg atgaatggcc agaaactttc cggtgtcgta     540
   agcgacaaca aaaaccagat tgaaaaaaac agtacggcga ttaccgaaca gcaggaacaa     600
   atcactgcca cgcaaaaaac cgttgccgcc actgctgatg cgcaacaatc acagtctgaa     660
   gaccaacaaa cgcagattac agagcaacag aagcagatcg cagccacaga aaaaacggtc     720
   gctgcaatcg gcgatcccca gtcaactgcg cattatcaaa agatgattaa tgccaaactg     780
   acggcgcaaa atgaagccaa tgaacgttca accgctgatc aggagcagag aattaatgaa     840
   ctggcttcca atgtcgcgac gcaacagcaa atcgatagcg tgcaatacgg tgaacaaatt     900
   cgaaaccttg cgcaagactc ggcccaggca catgagcaaa tcgaaagtct gacacaagat     960
   tctgcccaaa cgcaccagca gttaacaaac acgcaaaaac gtgttgcaga taacagccag    1020
   caaatcaatt cgctcaataa caacttcagt tcgttgaaac acgaagtgga agataaccgt    1080
   aaagaggcta atgcgggtat tgcgtcagcc gtcgctattg cttcacagcc tcaagttaag    1140
   acaggtgatt ttatgatggt gtctgccggg gcgggtacat tcaacaatga atctgcggta    1200
   tctgtcggtg cttcgtttaa tgctggaatt catacggtaa ttaaagcggg agtctctgcg    1260
   gatacacaat ctgatttcgg cgcgggtgtc ggcgtggggt attcgttcta a             1311
<210> 2
   <211> 436
   <212> PRT
   <213> Escherichia coli
<220>
   <221> misc_feature
   <223> Adhésine/invasine putative IHP1-like
<400> 2
   Met Lys Ser Val Lys Leu Ser Leu Leu Ala Val Val Val Ala Thr Ile
   1 5 10 15
   Val Ser Pro Ser Ala Phe Ala Asp Asp Thr Val Glu Ala Ala Ala Thr
   20 25 30
   Glu Leu Thr Ala Ile His Pro Gly Met Ser His Asp Glu Ile Asp Gln
   35 40 45
   Lys Ile Asn Arg Phe Met Ile Arg Thr Gly Asn Thr Glu Ala Ala Gln
   50 55 60
   Asp Tyr Leu Phe Ser His Gly Tyr Met Asp Pro Gln Thr Gly Asp Asn
   65 70 75 80
   Ile Tyr Pro Thr Ser Thr Ala Val Gln Thr Asp Ser Thr Val Thr Asp
   85 90 95
   Lys Asn Gln Ala Asp Arg Asp Ala Ala Gln Asp Thr Ala Ile Gln Asn
   100 105 110
   Ala His Asn Ile Ala Ser Ile Ala Ala Ala Asn Ala Asp Asp Ala Lys
   115 120 125
   Lys Ala Leu Thr Thr Ala Gln Ser Ala Ile Asp Ala Asn Lys Gly Ala
   130 135 140
   Ile Ala Asp Thr Gln Lys Asp Val Ala Asp Val Lys Ala Ser Ala Asp
   145 150 155 160
   His Ala Asn Glu Asn Ala Asn Thr Ala Leu Met Asn Gly Gln Lys Leu
   165 170 175
   Ser Gly Val Val Ser Asp Asn Lys Asn Gln Ile Glu Lys Asn Ser Thr
   180 185 190
   Ala Ile Thr Glu Gln Gln Glu Gln Ile Thr Ala Thr Gln Lys Thr Val
   195 200 205
   Ala Ala Thr Ala Asp Ala Gln Gln Ser Gln Ser Glu Asp Gln Gln Thr
   210 215 220
   Gln Ile Thr Glu Gln Gln Lys Gln Ile Ala Ala Thr Glu Lys Thr Val
   225 230 235 240
   Ala Ala Ile Gly Asp Pro Gln Ser Thr Ala His Tyr Gln Lys Met Ile
   245 250 255
   Asn Ala Lys Leu Thr Ala Gln Asn Glu Ala Asn Glu Arg Ser Thr Ala
   260 265 270
   Asp Gln Glu Gln Arg Ile Asn Glu Leu Ala Ser Asn Val Ala Thr Gln
   275 280 285
   Gln Gln Ile Asp Ser Val Gln Tyr Gly Glu Gln Ile Arg Asn Leu Ala
   290 295 300
   Gln Asp Ser Ala Gln Ala His Glu Gln Ile Glu Ser Leu Thr Gln Asp
   305 310 315 320
   Ser Ala Gln Thr His Gln Gln Leu Thr Asn Thr Gln Lys Arg Val Ala
   325 330 335
   Asp Asn Ser Gln Gln Ile Asn Ser Leu Asn Asn Asn Phe Ser Ser Leu
   340 345 350
   Lys His Glu Val Glu Asp Asn Arg Lys Glu Ala Asn Ala Gly Ile Ala
   355 360 365
   Ser Ala Val Ala Ile Ala Ser Gln Pro Gln Val Lys Thr Gly Asp Phe
   370 375 380
   Met Met Val Ser Ala Gly Ala Gly Thr Phe Asn Asn Glu Ser Ala Val
   385 390 395 400
   Ser Val Gly Ala Ser Phe Asn Ala Gly Ile His Thr Val Ile Lys Ala
   405 410 415
   Gly Val Ser Ala Asp Thr Gln Ser Asp Phe Gly Ala Gly Val Gly Val
   420 425 430
   Gly Tyr Ser Phe
   435
<210> 3
   <211> 423
   <212> DNA
   <213> Escherichia coli
<220>
   <221> misc_feature
   <223> ORF2 du locus SILO145
<400> 3
   atgatgaata gctccattaa atcgttttcc ctgctggcgg ttatattact ggctggctgt      60
   agttcaccca cttcccgcat cgcagattgc caggcgcagg gcgtcagtca tgacacctgt     120
   tacctcgcag aacagcagcg tcaggcggct attttaagtg catccgaggc acaggcattt     180
   aaaaatgcag aagccgcaca acacgcccag gcggcaaaga aagccattta taaaggattt     240
   ggcatgacct ttagaatgag cagtaaaaac tttgcttatc tcaatgattc attatgtgca     300
   attgatgaag acaataaaga tgccactgtt tatcagtcag gtctgtataa cgtcattgtt     360
   tatcatcaca caggaaaagt cgccttaatg aaagaaggcc agttcgtggg ttatttaaaa     420
   tga                                                                    423
<210> 4
   <211> 140
   <212> PRT
   <213> Escherichia coli
<220>
   <221> misc_feature
   <223> Protéine IHP2
<400> 4
   Met Met Asn Ser Ser Ile Lys Ser Phe Ser Leu Leu Ala Val Ile Leu
   1               5                   10                  15
   Leu Ala Gly Cys Ser Ser Pro Thr Ser Arg Ile Ala Asp Cys Gln Ala
               20                  25                  30
   Gln Gly Val Ser His Asp Thr Cys Tyr Leu Ala Glu Gln Gln Arg Gln
           35                  40                  45
   Ala Ala Ile Leu Ser Ala Ser Glu Ala Gln Ala Phe Lys Asn Ala Glu
       50                  55                  60
   Ala Ala Gln His Ala Gln Ala Ala Lys Lys Ala Ile Tyr Lys Gly Phe
   65                  70                  75                  80
   Gly Met Thr Phe Arg Met Ser Ser Lys Asn Phe Ala Tyr Leu Asn Asp
                   85                  90                  95
   Ser Leu Cys Ala Ile Asp Glu Asp Asn Lys Asp Ala Thr Val Tyr Gln
               100                 105                 110
   Ser Gly Leu Tyr Asn Val Ile Val Tyr His His Thr Gly Lys Val Ala
           115                 120                 125
   Leu Met Lys Glu Gly Gln Phe Val Gly Tyr Leu Lys
       130                 135                 140
<210> 5
   <211> 363
   <212> DNA
   <213> Escherichia coli
<220>
   <221> misc_feature
   <223> Complémentaire de l'ORF3 du locus SILO145
<400> 5
   ttactcttcc cggcctttac tcatactttt ttggtcttca tccggatagt gtttttttag     60
   atattccagg acgtttttat tgaccttgtg ttgggtatac acccaccctt tccagtaatc    120
   aggctggtcc aggtaaactt ctggcggaat ggtgaaatca gaaagcgtta accattcggc    180
   taacagatcg gggtttcgtt tctggatcaa ctgcaacagc ataatcagcg acatggcaga    240
   ggcaggagcc gtactatcgc cgcttaaata cttccacact gtcgaccggt tcgaacgaaa    300
   aaggatcggt agcaatgccc ggtccagatt catttcatta aaaatcttct caaattcatt    360
   cat                                                                  363
<210> 6
   <211> 120
   <212> PRT
   <213> Escherichia coli
<220>
   <221> misc_feature
   <223> Protéine IHP3
<400> 6
   Met Asn Glu Phe Glu Lys Ile Phe Asn Glu Met Asn Leu Asp Arg Ala
   1               5                   10                  15
   Leu Leu Pro Ile Leu Phe Arg Ser Asn Arg Ser Thr Val Trp Lys Tyr
               20                  25                  30
   Leu Ser Gly Asp Ser Thr Ala Pro Ala Ser Ala Met Ser Leu Ile Met
           35                  40                  45
   Leu Leu Gln Leu Ile Gln Lys Arg Asn Pro Asp Leu Leu Ala Glu Trp
       50                  55                  60
   Leu Thr Leu Ser Asp Phe Thr Ile Pro Pro Glu Val Tyr Leu Asp Gln
   65                  70                  75                  80
   Pro Asp Tyr Trp Lys Gly Trp Val Tyr Thr Gln His Lys Val Asn Lys
                   85                  90                  95
   Asn Val Leu Glu Tyr Leu Lys Lys His Tyr Pro Asp Glu Asp Gln Lys
               100                 105                 110
   Ser Met Ser Lys Gly Arg Glu Glu
           115                 120
<210> 7
   <211> 1204
   <212> DNA
   <213> Escherichia coli
<220>
   <221> misc_feature
   <223> ORF4 du locus SILO145
<400> 7
   atgctttttc cctttcgaaa acctgagtac gtctgctgaa aaataccgct gcgggcgtca    60
   ttttttcggt gaagtccggt aaaggtttct gcgtcaatgt gtgctttacg atccgcaccc   120
   ttatgggctt atccgttcac tctgtggcag gatgtcccgg taatacgttt ctggtccgag   180
   gccggctgcc ccacctgcgc ggatttgtct acagcggttt tgcggatgat gaacagggag   240
   ccgcccggtt tttgtctgct ctgaaaactg gaatcgccca tggtctggct ttacggacgc   300
   ttttgccgcg cttacaccgc tttctcatgc cttgctgatg gctattatct gctggaagat 360
   cgtgaacttt acccgacgga ggcaatgggc atttcttttg ggcggcaacg gaccatagcg 420
   ccagtaatcc ggcaaccgtg ccgtctggga tcctgaatat tgctatttct gtaacaccgc 480
   cccctgtttt cttcttccgg acagccgcca tcacatttta atcctgagcg tgtgacgttt 540
   tatcgcgata aaccggagcc cgcgctttgg cctggtatct ccctgattcc tatctctgcg 600
   ttctgctcga cgggcacata aggccactgc tgccgcactg gaagggcgac cgctcaaaac 660
   actggtactc agcacgcaac ccgttttaat gatgagcaac aaaccctggt atttccgggc 720
   ggagaatgtc tgcaaaaaca gagctactgt gccacgttcc caaactcact gagtggaaaa 780
   cattaccgtc cgggcatggg agagtttcgg ccctgataag cacatcagcc catccgaaac 840
   ctggtcagaa gattgcagca gagcgtcagc cggtaccctt cactggatca agcctggcaa 900
   attgttgagg cgggaatttg agcgaaactc gtattaagag catgatccaa caaggtttgg 960
   gggaggatga aaggctgatg taattttgca ggcgctgttt ttcacacact cgccgctttt 1020
   tattgacttg cccgattcgt tatcagctat ccggcatacg tcagttatcg ccccctcacc 1080
   ttccggttat ggcgcaaaac cgaacgccgc aagcagatgc tttttttctg gattttgcca 1140
   tcaatgagac ggcgaacgtc cggagttgac gaaaataatg gacgactatt ttcgcaagcg 1200
   ttaa 1204
<210> 8
   <211> 407
   <212> PRT
   <213> Escherichia coli
<220>
   <221> misc_feature
   <223> Protéine cytoplasmique putative
<400> 8
   Met Leu Phe Pro Phe Arg Lys Pro Glu Val Arg Leu Leu Lys Asn Thr
   1 5 10 15
   Ala Ala Gly Val Ile Phe Ser Val Lys Ser Gly Lys Gly Phe Leu Arg
   20 25 30
   Gln Cys Val Leu Tyr Asp Pro His Pro Tyr Gly Leu Ile Arg Ser Leu
   35 40 45
   Cys Trp Gln Asp Val Pro Val Ile Arg Phe Trp Ser Glu Ala Gly Cys
   50 55 60
   Pro Thr Cys Ala Glu Phe Val Tyr Ser Gly Phe Ala Asp Asp Glu Gln
   65 70 75 80
   Gly Ala Ala Arg Phe Leu Ser Ala Leu Glu Asn Trp Asn Arg Pro Trp
   85 90 95
   Ser Gly Phe Thr Asp Ala Phe Ala Ala Leu Thr Pro Leu Phe Ser Cys
   100 105 110
   Leu Ala Asp Gly Tyr Tyr Leu Leu Glu Asp Arg Glu Leu Tyr Pro Thr
   115 120 125
   Asp Gly Asn Gly His Phe Phe Trp Ala Ala Thr Asp His Ser Ala Ser
   130 135 140
   Asn Pro Ala Thr Val Ala Val Trp Asp Pro Glu Tyr Cys Tyr Phe Cys
   145 150 155 160
   Asn Thr Ala Pro Cys Phe Leu Leu Pro Gly Gln Pro Pro Ser His Phe
   165 170 175
   Asn Pro Glu Arg Val Thr Phe Tyr Arg Asp Lys Pro Asp Ala Arg Ala
   180 185 190
   Leu Ala Trp Tyr Leu Pro Asp Ser Tyr Leu Cys Val Leu Leu Asp Gly
   195 200 205
   His His Lys Ala Thr Ala Ala Ala Leu Glu Gly Arg Pro Leu Lys Thr
   210 215 220
   Leu Val Leu Ser Thr Ala Thr Arg Phe Asn Asp Glu Gln Gln Thr Leu
   225 230 235 240
   Val Phe Pro Gly Gly Glu Cys Leu His Lys Thr Glu Leu Leu Cys His
   245 250 255
   Val Pro Lys Leu Thr Glu Trp Lys Thr Leu Pro Ser Gly Ala Trp Glu
   260 265 270
   Ser Phe Gly Pro Asp Lys His Ile Ser Pro Ser Glu Thr Trp Ser Glu
   275 280 285
   Glu Leu Gln Gln Ser Val Ser Arg Tyr Pro Ser Leu Asp Gln Ala Trp
   290 295 300
   Gln Ile Val Glu Ala Gly Asn Leu Ser Glu Thr Arg Ile Lys Ser Met
   305 310 315 320
   Ile Gln Gln Gly Leu Gly Glu Asp Glu Lys Ala Asp Val Ile Leu Gln
   325 330 335
   Ala Leu Phe Phe Thr His Ser Pro Leu Phe Ile Asp Phe Ala Arg Phe
   340 345 350
   Val Ile Ser Tyr Pro Ala Tyr Val Ser Tyr Arg Pro Leu Thr Phe Arg
   355 360 365
   Leu Met Ala Gln Asn Arg Thr Pro Gln Ala Asp Ala Phe Phe Leu Asp
   370 375 380
   Phe Ala Ile Asn Asp Asp Gly Glu Arg Pro Glu Leu Thr Lys Ile Met
   385 390 395 400
   Asp Asp Tyr Phe Arg Lys Arg
   405
<210> 9
   <211> 366
   <212> DNA
   <213> Escherichia coli
<220>
   <221> misc_feature
   <223> Fragment de 366 pb de l'ORFI du locus SILO145
<400> 9
   ggcgataata tttaccccac cagtacagcc gtacagactg acagcactgt caccgataaa     60
   aaccaggcag atcgcgacgc ggcgcaagat accgccattc agaatgcaca caatatcgca    120
   agcattgcgg cggcaaatgc agatgacgcc aaaaaagcgc ttaccactgc gcaatctgca    180
   attgatgcga acaagggagc tattgctgat actcagaaag atgtcgcaga tgttaaagca    240
   agtgcagatc atgccaacga gaacgctaat accgcgttga tgaatggcca gaaactttcc    300
   ggtgtcgtaa gcgacaacaa aaaccagatt gaaaaaaaca gtacggcgat taccgaacag    360
   caggaa                                                               366
<210> 10
   <211> 132
   <212> DNA
   <213> Escherichia coli
<220>
   <221> misc_feature
   <223> Fragment de 132 pb de l'ORF1 du locus SILO145, amplifié par les amorces O145F268 et O145R399
<400> 10
   cgataatatt taccccacca gtacagccgt acagactgac agcactgtca ccgataaaaa     60
   ccaggcagat cgcgacgcgg cgcaagatac cgccattcag aatgcacaca atatcgcaag    120
   cattgcggcg gc                                                        132
<210> 11
   <211> 26
   <212> DNA
   <213> Artificial sequence
<220>
   <223> Amorce PCR: O145F268
<400> 11
   cgataatatt taccccacca gtacag 26
<210> 12
   <211> 15
   <212> DNA
   <213> Artificial sequence
<220>
   <223> Amorce PCR: O145R399
<400> 12
   gccgccgcaa tgctt 15
<210> 13
   <211> 27
   <212> DNA
   <213> Artificial sequence
<220>
   <223> Sonde interne TaqMan: O145TQ357
<400> 13
   ccgccattca gaatgcacac aatatcg 27
<210> 14
   <211> 26
   <212> DNA
   <213> Artificial sequence
<220>
   <223> Amorce PCR: LJL1
<400> 14
   cgctgctcgc cgcctggctg tggtgg 26
<210> 15
   <211> 25
   <212> DNA
   <213> Artificial sequence
<220>
   <223> Amorce PCR: RJP4
<400> 15
   gaacgatgaa catcagcgat gtagc                                              25